# EUROPEAN PATENT APPLICATION

(11) **EP 2 003 452 A1**
(43) Date of publication of application: **17.12.2008**
(21) Application number: 07450106.5
(22) Date of filing: 11.06.2007
(51) Int. Cl.: G01N 33/543

(54) **Method for diagnosing an allergy**

(71) Applicant: Universität für Bodenkultur Wien, 1180 Wien (AT)
(72) Inventor: Altmann, Friedrich, 1190 Vienna (AT); Chungsheng, Jin, 1190 Vienna (AT)
(74) Representative: Sonn & Partner Patentanwälte

(57) **Abstract**

The present invention relates to a method for removing cross-reactive carbohydrate determinant (CCD) specific antibodies from a sample comprising the steps of:
- providing a sample comprising or suspected to comprise CCD specific antibodies,
- contacting and incubating said sample with a solid support comprising at least one cross-reactive carbohydrate determinant, immobilised on its surface, and
- removing said solid support from the sample.

## Description

The present invention relates to a method for removing cross-reactive carbohydrate determinant (CCD) specific antibodies from a sample.

Carbohydrate cross-reactive determinants (CCDs) are carbohydrate moieties of glycoproteins that induce the production of highly cross-reactive IgE. Many allergens are glycoproteins containing carbohydrate moieties (N-glycans or O-glycans depending to their site of attachment to the protein). Till today N-glycans containing β1,2-xylose and α1,3-fucose have been found in many glycoproteins. Despite the great diversity of carbohydrate structures, some common patterns are present in glycoproteins of invertebrate animals and plants.

In the early 1980s, the presence of IgE directed against these carbohydrate determinants was reported in patient sera. The production of such IgE antibodies could result from exposure to allergens in the form of pollen or from an insect sting.

Exposure to pollens seems to be an important cause of the production of IgE that cross-reacts with carbohydrate determinants. Carbohydrate determinants have been described in tree and weed pollens, but the major elicitor of anti-CCD IgE seems to be grass pollen.

Anti-CCD IgE has also been observed in response to several vegetables, fruits and seeds, like tomato, apple, peanut, hazelnut.

Cross-reactions between pollens and plant-derived foods are often caused by CCDs, because the CCD structures are identical.

The hypothesis that anti-CCD IgE has no clinical relevance predominates because the presence of anti-CCD IgE continues to be associated with negative reactions to skin prick tests and lack of clinical symptoms. Because many glycoproteins carry only one IgE-binding glycan, they would be unable to cross-link IgE bound to the receptors of the mast cells and basophils for reasons related to epitope accessibility and antibody affinity; thus clinical symptoms might not appear in patients whose IgE response is restricted to CCDs. Nevertheless, it has been reported that anti-CCD IgE can be detected as such by *in vitro* tests. CCDs should be considered a potential cause of interference in these tests. Data on the prevalence and distribution of anti-CCD IgE as well as anti-CCD IgG prevalence in a large cohort of subjects with allergy symptoms are limited.

When a patient with anti-CCD IgE is tested for a certain allergen, for example, by an *in vitro* method, the potential interference by CCD can have several effects. If the patient is sensitised to said allergen but the assay detects IgE directed against both the protein and the carbohydrate structures of said allergen, positive test results for this allergen will be exaggerated. If the patient is not sensitised to said allergen but the assay detects anti-CCD IgE, test results for this allergen will result in a false positive diagnosis. Such misdiagnosis could result in unnecessary avoidance and/or inappropriate immunotherapy, which may even pose a risk of inducing a new allergy.

It is possible to prove the presence of anti-CCD IgE in patient serum by evaluating the IgE reactivity against well-characterised glycoproteins. The use of such glycoproteins as an anti-CCD IgE "marker" can indicate the presence in serum of IgE directed against carbohydrate epitopes. The glycoprotein most widely used to identify IgE reactivity to carbohydrate determinants is the pineapple protease bromelain. It is known to be a good marker because its carbohydrate chain is very similar to those of many other plant proteins and because a true allergy to bromelain is very rare. Bromelain can be used as an inhibitor in quantitative or qualitative competitive inhibition measurements. Inhibition procedures are well-standardised procedures to distinguish between cross-reactivity and true double sensitisation. An elevated result for a bromelain-specific IgE test suggests normally the presence of anti-CCD IgE in the sample.

Conventional serum-based assays, such as ImmunoCAP^{™} and other allergen specific IgE tests, are at risk of carbohydrate interference. But this also applies for immunoblotting assays.

It is an object of the present invention to provide an accurate and fast method for diagnosing a specific allergy in a patient avoiding cross reactivity with other allergens. A further object of the present invention is providing a method for removing CCD specific antibodies from a sample.

Therefore, the present invention relates to a method for removing cross-reactive carbohydrate determinant (CCD) specific antibodies from a sample comprising the steps of:
- providing a sample comprising or suspected to comprise CCD specific antibodies,
- contacting and incubating said sample with a solid support comprising at least one cross-reactive carbohydrate determinant immobilised on its surface, and
- removing said solid support from the sample.

The method of the present invention allows to remove efficiently CCD specific antibodies from a sample. This removal is achieved by contacting the sample with a solid support having immobilised on its surface at least one cross-reactive carbohydrate determinant. The removal of CCD specific antibodies from a sample is particularly advantageous when the sensitivity of a patient to certain allergen is analysed. Allergens having N- or O-glycans bound to them are able to induce in an individual the formation of antibodies directed to these glycan structures. Since the glycan structures are present in many different allergens, the presence of CCD specific antibodies in a sample obtained from an individual may lead to false positive results and consequently to a false diagnosis (CCD's mask allergy diagnosis). Therefore it is advantageous to remove prior such test assays CCD specific antibodies from the sample to be analysed, so that preferably only protein/polypeptide specific antibodies remain in the sample.

The CCD may be immobilised on the surface of the solid support via at least one amino acid residue, which is preferably asparagine, which represents the natural amino acid residue to which CCDs usually are bound. However, it is of course possible to use another molecule or amino acid residue as linker, provided that said molecule or amino acid residue does not change the conformation or structural properties of the CCD or its linking sugar residue. Such a change can be determined by performing binding assays of CCD specific antibodies to said altered linking. If CCD specific antibodies still bind to the CCD bound to another molecule than asparagine said molecule may be used as an alternative linker. Furthermore, said molecule or asparagine, acting as a linker, may be part of a peptide, polypeptide or even protein, provided that said peptide, polypeptide or protein does not comprise allergen (T-cell or B-cell epitopes). In such a case also antibodies may be removed from the sample which are specific for the proteinaceous portion of an allergen. This will lead to a false negative result, if the sample obtained by the method of the present invention is used to determine the presence of antibodies directed to an allergen. Therefore the linker or the CCD is preferably bound only to one amino acid residue (preferably asparagine), which itself is bound to a maximum of one, preferably a maximum of two, three, four, five, ten, 15, 20, 30, 50, amino acid residues provided that these amino acid residues do not comprise an allergen-specific T-cell or B-cell epitope.

As used herein, the term "carbohydrate cross-reactive determinant" or "CCD" relates to glycan structures of proteins, in particular allergens derived from plants and insects. CCDs are able to provoke a specific immune response in a mammal. CCDs are usually asparagine-linked oligosaccharides (N-glycan) and contain core α1,3-linked fucose and/or β1,2-xylose. It is noted that in contrast to this plant/insect CCD, mammalian glycoproteins may contain a mammalian CCD comprised of the sugar sequence Galα1-3Galβ1-4GlcNAcβ1- that is immunogenic in humans (Galili U. Immunol. Cell. Biol. 83 (2005): 674-686) and against which some allergic patients have IgE antibodies. Yet another type of CCD occurs on some plant allergens, certainly on the mugwort pollen protein Art v 1 in the form of single β-arabino-furanosyl residues linked to hydroxyprolines (Léonard R. et al., J. Biol. Chem. 280 (2005): 7932-7940).

According to a preferred embodiment of the present invention the sample comprises allergen specific antibodies.

The sample from which the CCD specific antibodies are removed comprises preferably allergen specific antibodies.

According to another preferred embodiment of the present invention the allergen specific antibodies are of the immunoglobulin isotype IgE, IgA and/or IgG.

After removal of the CCD specific antibodies from the sample the specificity and/or amount of the allergen specific antibodies present in the sample is preferably determined. Due to said removal cross reactivity between antibodies and various allergens can be avoided and only antibodies which are specific for the proteinaceous moiety of the allergen are detected,

Another aspect of the present invention relates to a method for diagnosing an allergy in an individual comprising the steps of:
- providing a sample of an individual,
- removing cross-reactive carbohydrate determinant (CCD) specific antibodies from said sample by contacting said sample and incubating said sample with a solid support comprising at least one cross-reactive carbohydrate determinant, immobilised on its surface, and removing said solid support from the sample,
- determining the presence and/or the amount of IgE specific for at least one allergen in the sample and
- diagnosing an allergy when the presence of allergen specific IgE is determined in the sample.

The removal of CCD specific antibodies from a sample allows, if the sample is obtained from an individual, to determine the presence of IgE molecules specifically binding to the proteinaceous moiety of an allergen. Methods for the qualitative and quantitative detection of allergen specific IgE's are well known in the art (e.g. ImmunoCAP^{®}, radioallergosorbent test (RAST), multiple radioallergosorbent test (MAST), fluorescent allergosorbent test (FAST), paper radioimmunosorbent test (PRIST), radioimmunosorbent test (RIST), enzyme-linked immunosorbent assay (ELISA)).

According to a preferred embodiment of the present invention the sample is of human or animal origin and preferably selected from the group consisting of whole blood, serum and plasma.

The method of the present invention may be performed with any kind of sample, provided that said sample comprises or is suspected to comprise CCD specific antibodies. A sample suspected to comprise CCD specific antibodies is a sample from which is not exactly known whether said sample comprises such antibodies. Therefore the sample is preferably obtained from a patient who is suspected to be allergic against a certain allergen or which is known to be sensitive for an allergen.

According to a preferred embodiment of the present invention the CCD is selected from the group consisting of β1,2-xylose and α1,3-fucose and is preferably bound to a further sugar moiety comprising or containing Manp(β1,4)-GlcpNAc(β1,4)-GlcpNAc(β1,N) resulting in a glycan selected from the group consisting of (Manα1-3)Manα1-6(Xylβ1-2)Manβ1-4GlcNAcβ1-4GlcNAc (MMX), (Manα1-3)Manα1-6(Xylβ1-2)Manβ1-4GlcNAcβ1-4(Fucα1-3)GlcNAc (MMXF), Manα1-6(Xylβ1-2)Manβ1-4GlcNAcβ1-4(Fucα1-3)GlcNAc (MUXF), Manα1-6(Mancα1-3)Manβ1-4GlcNAcβ1-4 (Fucα1-3)GlcNAc (MMF), Manα1-6Manβ1-4GlcNAcβ1-4(Fucα1-3)GlcNAc (MUF), GlcNAcβ1-2Manα1-6(GlcNAcβ1-2Manα1-3) (Xylβ1-2)Manβ1-4GlcNAcβ1-4 (Fucα1-3) GlcNAc (GnGnXF), GlcNAcβ1-2Manα1-6 (Manα1-3)(Xylβ1-2)Manβ1-4GlcNAcβ1-4(Fucα1-3)GlcNAc (GnMXF), Manα1-6 (GlcNAcβ1-2Manα1-3) (Xylβ1-2) Manβ1-4GlcNAcβ1-4(Fucα1-3)GlcNAc (MGnXF), GlcNAcβ1-4Manα1-6(Manα1-3)Manβ1-4GlcNAcβ1-4 (Fucα1-3) GlcNAc (GnMF), Manα1-6(GlcNAcβ1-2Manα1-3)GlcNAcβ1-4(Fucα1-3)GlcNAc (MGnF) or GlcNAcβ1-2Manα1-6(GlcNAcβ1-2Manα1-3)Manα1-4GlcNAcβ1-4(Fucα1-3)GlcNAc (GnGnF).

The glycan or CCD which is immobilised on a solid carrier is preferably isolated from a plant or mammalian glycoprotein. Due to the natural occurrence of CCD structures it is particularly advantageous to obtain the sugar moieties to be immobilised from a natural source. Therefore, fragments of the glycoprotein comprising the glycan or CCD or entire glycoprotein may be used for immobilisation. However, it should be noted that the proteinaceous portion of the isolated plant or mammalian glycoprotein does not show any, or shows only a very reduced cross reactivity with the allergens analysed. The CCD may also be coupled to a proteinaceous or non-proteinaceous carrier molecule via an in vitro reaction known in the state of the art and described exemplarily in the example section under "preparation of glycan allergens".

The glycan or CCD may further be synthesized chemically with methods known in the art. Furthermore, it is of course also possible to synthesize then *in vitro* with corresponding enzymes which catalyse the formation of glycan structures.

According to a preferred embodiment of the present invention the plant glycoprotein is obtained from bromelain, patatin, horseradish peroxidase, or other plant glycoproteins, which are not significant allergens based on their protein-moiety (such as phospholipase A), and which have a defined glycan structure.

According to another preferred embodiment of the present invention the mammalian glycoprotein is obtained from fibrin and/or other vertebrate glycoproteins containing a substantial amount of diantennary N-glycans (e.g. serotransferrin, protease-inhibitors, immunoglobulins or egg yolk glycoproteins) is preferably modified with glycosidases or glycosyl transferases.

If the glycoprotein used according to the present invention does not comprise all structural features of the CCD's the glycan structure of such proteins may be modified accordingly by using glycosidases and/or glycosyl transferases.

According to a preferred embodiment of the present invention the solid support is a conventional affinity matrix consisting of a matrix such as porous beads, monolithic chromatography bed or a functionalised permeable membrane, functionalised or activated cellulose, agarose, dextran, poly-methacrylate, polystyrol, polyacrylamide or silica.

According to the present invention it is also possible to immobilise mammalian glycans or CCDs on a carrier. Such CCDs may be Galα1-3Galβ1-4GlcNAcβ1-. These α-Gal CCDs occur on many glycoproteins of mammals but not of man. They may be isolated from natural glycoproteins similar as plant/insect CCDs, e.g. from calf intestine phosphatase (Bublitz et al., J. Mass Spectrom. 36 (2001): 960-972), or synthesised with the help of α1,3-galcto-syl-transferase on desialylated mammalian serum glycoproteins (e.g. asialo-forms of fetuin, α1-acid glycoprotein, Tamm-Horse-fall glycoprotein, serotransferrin, protease-inhibitor, immunoglobulins, egg yolk glycoproteins or other glycoproteins with preferably known N-glycans structures lacking interfering determinants), glycopeptides or oligosaccharides derived thereof. Alternatively, the determinant may also be fully or partially synthesised chemically. A solid support carrying this CCD would help to eliminate antibodies against this mammalian CCD, which in some allergic patients may lead to false positive results with animal allergens.

Another aspect of the present invention relates to the use of a solid support comprising at least one cross-reactive carbohydrate determinant, immobilised on its surface, in diagnosing an allergen sensitivity.

Solid supports having on their surface immobilised CCD may be suitably employed in allergy diagnosis. Prior to diagnose an allergy sensitivity in an individual CCD specific antibodies are removed and allergen specific antibodies are determined in the sample which is preferably blood, plasma, serum or nasal fluid. The determination of allergen specific antibodies may be performed with methods known in the art.

Yet another aspect of the present invention relates to a kit for diagnosing an allergen sensitivity comprising:
- a solid support comprising at least one cross-reactive carbohydrate determinant, immobilised on its surface,
- means for determining the presence of allergen specific antibodies.

According to a preferred embodiment of the present invention the means for determining the presence of allergen specific antibodies comprise an allergen and means for detecting and optionally quantifying the binding of the allergen specific antibodies to the allergen.

Means for determining qualitatively and quantitatively the binding of an allergen specific antibody to an allergen are well known in the art (e.g. ImmunoCAP^{®}, radioallergosorbent test (RAST), multiple radioallergosorbent test (MAST), fluorescent allergosorbent test (FAST), paper radioimmunosorbent test (PRIST), radioimmunosorbent test (RIST), enzyme-linked immunosorbent assay (ELISA)).

The present invention is further illustrated by the following examples, however, without being restricted thereto.

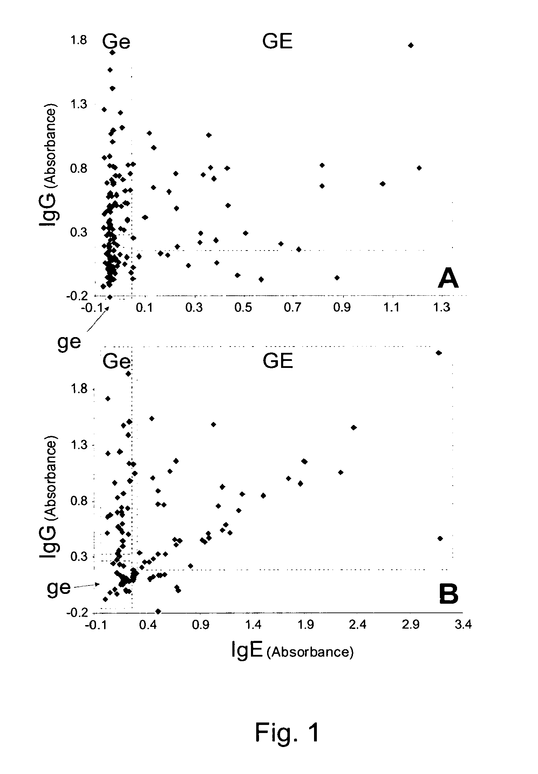

Fig. 1 shows a plot of levels of CCD-IgE versus that of CCD-IgG in sera of allergic patients. Comparison of ELISA readings of antibodies binding to a plant glycoprotein (HRP) serving as a CCD-allergen. Panel A shows the results obtained with the 170 sera from which those used for affinity measurement were selected. Panel B shows another group of 110 sera analyzed in the course of a recent study. The values are unstandardized absorbance readings of single measurements. Blank-correction leads to negative values in some cases. The areas marked by dashed boxes arbitrarily designate three major groups of sera: "ge" = neither IgG nor IgE positive, "Ge" (only IgG positive) or "GE" = both s-IgE and s-IgG positve.

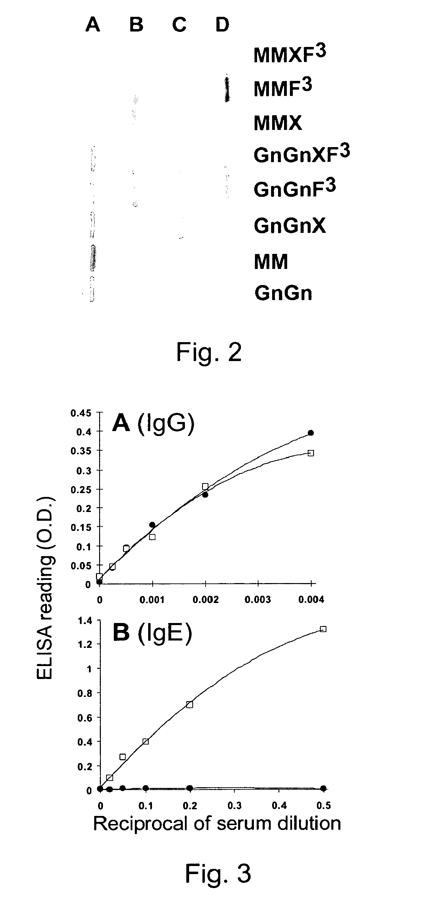

Fig. 2 shows binding of human IgG and IgE to various glycan structures. Equal amounts of various glycoforms of human transferrin were subjected to SDS-PAGE and either stained with Coomassie blue (lane A) or blotted onto nitrocellulose. Lane B was incubated with rabbit anti-HRP antiserum. Lane C shows the binding of IgG from the serum pool to virtually all glycoforms. Lane D demonstrates the strong preference of IgE from the pool for fucosylated glycoforms.

Fig. 3 shows the role of xylose for human IgG and IgE. Dilutions of the serum pool were incubated in ELISA plates with a plant-glycoprotein (HRP) containing xylose and fucose or with the snail glycoprotein hemocyanin containing xylose. The wells were stained either for bound IgG (panel A) or IgE (panel B). Values obtained for HRP are shown as open squares, for hemocyanin as black dots.

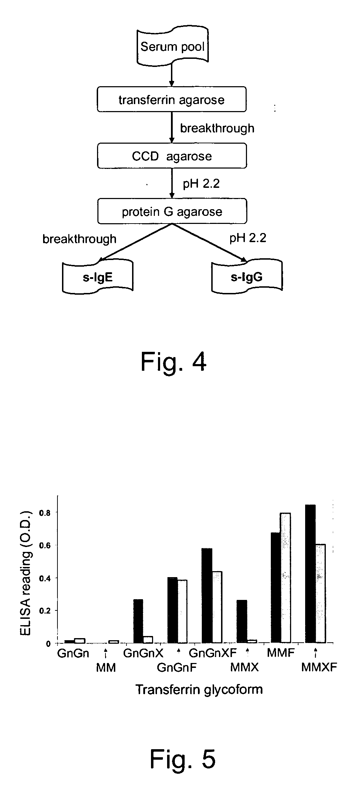

Fig. 4 shows the purification of specific IgE and IgG. The scheme depicts the steps of the antibody purification as described in the methods section. The flags symbolize serum or antibody preparations, the boxes chromatography columns. The CCD agarose contained a mixture of three BSA-glycopeptides conjugates. The glycopeptides were prepared from fibrin and were glycomodified to obtain MMX, MMF and MMXF-glycans.

Fig. 5 shows the binding specificity of purified human anti-MMXF IgG and IgE. ELISA plate wells were coated with different glycomodified transferrins. The black bars indicate the binding of human IgG to different glycoforms; the grey bars that of human IgE. Blank values, which were quite high in the case of IgG, were substracted.

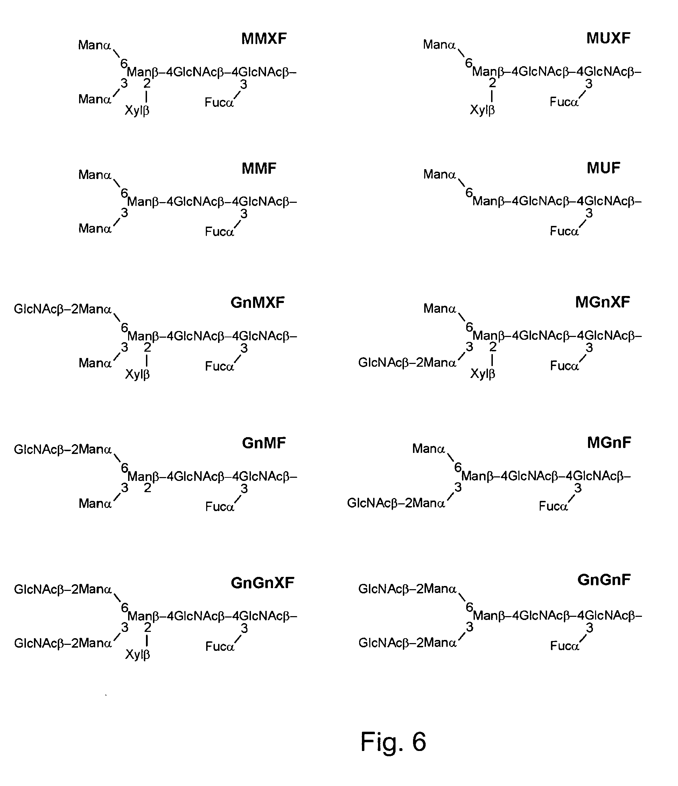

Fig. 6 shows the glycan molecules comprising the CCDs of the present invention.

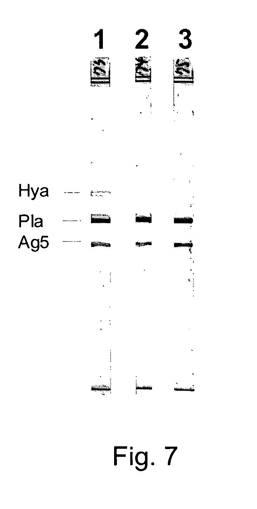

Fig. 7 shows the efficiency of anti-CCD IgE removal from serum of a wasp-allergic patient.

### EXAMPLES:

### EXAMPLE 1

N-glycans of plant and insect glycoproteins are the most widely distributed group of pan-epitopes of relevance in allergy, but the clinical impact of these cross-reactive carbohydrate determinants (CCDs) is currently considered as negligable and therefore the binding of IgE to CCDs in *in vitro* diagnostic tests causes false-positive results. The key elements of CCDs are the core α1,3-fucose occuring in insect glycoproteins, or this fucose plus a β1,2-xylose in plant glycoproteins as reviewed recently. Anti-CCD IgE was found in 15 to 30% of sera of allergic patients. The frequent occurence of sensitization against CCDs stands opposite to their apparent inability to cause clinical manifestations. When 331 CCD-positive patients were skin-prick tested with the polyvalent glycoprotein horseradish peroxidase (HRP), 79% did not exhibit any reactions and the rest showed weak reactions whose allergic nature may even be questioned. Notably, these 21% did not react towards ascorbate oxidase with three potential glycosylation sites of which two are certainly occupied by MMXF type glycans. Similar negative results have been obtained in several other studies. However, it cannot be excluded that patients with at least mild symptoms upon exposure to CCD allergens exist. However, todays general picture is that anti-CCD IgE is ineffective in eliciting allergic reactions. This is surprising as several studies have shown the ability of anti-CCD IgE to trigger mediator release from granulocytes. It is a matter of debate whether or not the concentrations required for effector release are higher for glyco-allergens as compared to allergens with peptide epitopes. It appears, that studies where defined, pure glyco-allergens have been used, point at a roughly comparable potency of glycan and peptide epitopes.

All the more, the question for the reason behind the clinical irrelevance of anti-CCD antibodies remains unanswered. One reason for failure to trigger immune cell degranulation can be the inability of cross-linking of surface-bound IgE by glycoproteins which are monovalent. However, there are numerous plant /insect glycoproteins with several glycosylation sites. Truly polyvalent food glycoproteins might be tomato invertase (Lyc e 2, Q8RVW4), a tomato peroxidase (P15003) or zucchini ascorbate oxidase (P37064). In the absence of site-specific glycosylation analysis, the valency of these candidates remains, however, undefined in contrast to *Vespula vulgaris* hyaluronidase with two MMFF glycans and the latex allergen Hev b 4 with 7 glycosylated sites, which, however, are only partially occupied with CCD structures.

Such "paucivalent" glycoproteins may represent borderline cases with an only low ability to elicit physiological reactions. The only example of a convincingly polyvalent plant glycoprotein is HRP with its seven N-glycans all having CCD structures. The inability of HRP to provoke positive skin tests in about 80% of CCD-reactive patients is a clear indication for the clinical insignificance even of polyvalent glycoproteins. With the new tools for site-specific glycosylation analysis, we expect that more polyvalent glyco-allergens will soon be described.

A second explanation assumes low binding affinity of anti-CCD IgE. Low affinity was in fact observed for the interaction between glycoproteins and glycan-binding protein such as C-type lectin, siglecs, and galectins. The affinities of these lectins for their glycan ligands are typically low (*K_{D}* of micromolar to millimolar) and therefore it requires multivalent interactions to achieve a biological effect. However, this does not necessarily apply to human anti-carbohydrate antibodies. The notorious α-Gal epitope can cause hyperacute rejection in pig-to-primate xenotransplantation. The IC₅₀ value of 4 x 10⁻⁸ M for the binding of α-Gal-polyacrylamide to anti-α-Gal antibody or the *K_{D}* of 10⁻⁹ M for an anti-LPS antibody demonstrates the principal possibility of high binding affinity between glycan determinants and antibodies.

In a preceding study, it was found that the affinity of rabbit anti-CCD IgG towards glyco-modified human transferrin was in the low nanomolar range. In the present example, surface plasmon resonance was employed to determine the specificities and affinities of IgE and IgG from sera of allergic patients against a glycoprotein with core α1,3-fucose and β1,2-xylose.

### MATERIALS and METHODS

### Patients and sera

Sera were collected from patients undergoing routine allergy testing for inhalant or insect venom allergy. Patients with inhalant allergy were skin-prick tested with common inhalant allergens, including pollens (hazel, alder, birch, grass, rye, ash, plantain, nettle, mugwort, ragweed, oilseed rape, plane tree), house dust mites, animal danders (cat, dog, horse, guinea pig), molds (Cladosporium, Alternaria, Penicillum), and rubber latex (all Soluprick, ALK, Demark). Insect venom allergy was diagnosed by demonstration of specific serum IgE (CAP-FEIA, Phadia, Sweden) and partly confirmed by subsequent skin prick test. According to allergy test results the sera were sorted into several groups: one with narrow sensitization such as grass pollen only (n=21), birch pollen only (n=21), or wasp venom only (n=22); then a group with sensitization to threee or more different pollens (n=45), double-positive CAP to bee and jellow jacket venom (n=10); and finally a group with discrepant CAP and SPT results for rubber latex (n=35). Patients in this latter group had positive CAP but negative or just weakly positive skin prick test results to latex. It shall not be concealed that this categorization is somewhat arbitrary and that groups may overlap.

### Preparation of glyco-allergens

Glyco-modified human transferrins with N-glycans with or without coreα1,3-fucose, xylose or terminal GlcNAc (GnGn-, MM-, GnGnX-, GnGnF-, GnGnXF-, MMX-, MMF-, and MMXF-Tf) were prepared from human apo-transferrin (Sigma-Aldrich) as described (Bencurova M et al.,(Glycobiology 14 (2004): 457-466); Jin C et al., (Glycobiology 16 (2006): 349-357). Covalent conjugates of glycopeptides with bovine serum albumin (BSA) were prepared using 2,5-dinitro-1,4-difluoro benzene as linker and fibrin-glycopeptides previously modified by glycosidases and glycosyltransferases similarily as the transferrin-derivates. The BSA conjugates (MMX-, MMF-, and MMXF-BSA) were subjected to amino sugar analysis (Altmann, Anal. Biochem. 204 (1992): 215-219) and found to contain 2-3 mol glycopeptides per mol of BSA. No glycosyltransferases or glycosidases of plant origin were used for these preparations to exclude contamination of the substances with plant glycoproteins.

### Measurement of CCD-specific and of total IqE and IgG

Levels of anti-CCD antibodies were determined in sera from allergic patients (n=131) by ELISA with HRP as coat antigen. Nunc 96-well plates (Demark) were coated for 1 hr, at 37°C with HRP (Sigma-Aldrich) at a concentration of 5 µg/mL in bicarbonate buffer (50 mM bicarbonate, pH 9.6) followed by blocking with TTBS buffer (0.1 M Tris-HCl, pH 7.2, 0.1 M NaCl, 2.5 mM MgCl2, 0.05% Tween-20) containing 1% BSA for 1 hr at ambient temperature. For determination of human anti-CCD IgG, sera at a dilution of 1:1,000 and alkaline phosphatase (AKP) conjugated goat anti-human IgG (H+L) (Jackson ImmunoResearch, PA, USA) at a dilution of 1:2,000 were used. For human anti-CCD IgE measurement, human sera at a dilution of 1:10 and alkaline phosphatase-conjugated mouse anti-human IgE (mAb G7-26, BD Pharmingen, Heidelberg, Germany) at dilution of 1:500 were used. Purified antibodies, both IgG and IgE were diluted 1:100. Visualization was carried out with 50 µl of 0.1% p-nitrophenyl phosphate in 0.1 M diethanolamine, pH 9.8. The color reaction was stopped by the addition of 100 µl 1 M H₂SO₄. Plates were read immediately at 405 nm. As the blank values obtained with uncoated wells were quite high, particularly in case of the IgG determinations, results were judged as positive if the O.D. difference was >0.15 or if the HRP-well gave twice the reading of the control well.

Alternatively, anti-CCD IgE and IgG was quantitated with the CAP-FEIA system (Phadia , Uppsala, Sweden) with bromelain serving as glyco-allergen. Total IgE was likewise determined with the CAP-FEIA system. Total IgG was quantitated by the ImmunoCAP system (Phadia) with some modifications. The calibration curve used in this assay was based on six different IgG calibrators (International reference preparation 67/86 from World Health Organization) with various concentrations of 0.02, 0.04, 0.1, 0.3, 1.0 and 2.0 mg/L, respectively. To obtain the calibration curve, the calibrators were incubated with ImmunoCAP IgG/IgA Calibrator which coated with monoclonal mouse antibodies against IgG to capture IgG. To measure total IgG in serum and purified anti-CCD IgG, the samples were incubated with ImmunoCAP IgG/IgA Calibrator instead of antigen-specific ImmunoCAP, and the assay otherwise performed as for specific IgG measurement according to the manufacture's instructions.

### Purification of human anti-CCD IgE and IgG

Serum samples from 19 CCD-IgE positive patients were mixed to give an anti-CCD serum pool of about 20 mL. Human IgG unexpectedly also bound to human transferrin. Therefore, the first purification step consisted of an affinity column with human transferrin conjugated to Affi-Gel 15 (BioRad, Richmond, CA) according to the manufacturer's instructions. The serum pool was diluted with the same volume of TBS buffer (50 mM Tris-HCl, pH 7.4, 150 mM NaCl, 0.05% NaN₃, 1:1) and applied to 1 mL of transferrin-gel. The non-binding antibody fraction was collected. To capture human anti-CCD antibodies, an affinity column with immobilized BSA-conjugates was employed. In order to ensure binding of anti-CCD antibodies of differing fine-specificity, an equimolar mixture of MMX-, MMF-, and MMXF-BSA was coupled to Af-fi-Gel 15. After loading of the anti-Tf depleted serum onto 2 mL gel, the bound human anti-CCD antibodies were eluted with 0.2 M glycine-HCl, pH 2.2. The eluate was immediately neutralized by 100 µL 1 M Tris-HCl, pH 7.5 already present in the collection vials. Fractions containing anti-CCD antibodies were detected by ELISA. The pooled sample was concentrated and brought into TBS buffer by centrifuge filter devices with 10 kDa cut-off (Millipore, Vienna, Austria).

Finally, human anti-CCD IgG was separated from anti-CCD IgE with the help of protein G plus agarose (Calbiochem, Darmstadt, Germany). The breakthrough of this column contained the CCD-specific IgE. The bound anti-CCD IgG was eluted and neutralized as describe above. The breakthrough (mainly IgE) and eluate (IgG) were concentrated by centrifugal ultrafiltration, and finally brought into TBS buffer containing 10% glycerol. The antibodies were kept at -20°C until use.

### Specificities of human anti-CCD IgG and IgE

The specificities of human anti-CCD IgG and IgE were determined by ELISA and Western blot using different transferrin glycoforms (unmodified Tf, NaNa-, GnGn-, MM-, GnGnX-, GnGnF-, GnGnXF-, MMX-, MMF-, and MMXF-Tf), the natural, xylose-containing glycoprotein hemocyanin from *Helix pomatia* (Sigma-Aldrich) or HRP. All glycoproteins were coated to plates at a concentration of 5 µg/mL. Otherwise, the procedure described above was followed.

For Western blot analysis, different transferrin glycoforms (1 µg) were separated by SDS-PAGE and electroblotted to a nitrocellulose membrane by semi-dry blotting (Biorad, Richmond, CA, USA). Human sera and secondary antibodies were diluted as for ELISA measurements. Immunoblots were stained with 5-bromo-4-chloro-3-indolyl phosphate plus nitroblue tetrazolium (Sigma-Aldrich).

### Affinity analysis of human anti-CCD IgG

The affinity constants of human anti-MMXF IgG and IgE were determined by surface plasmon resonance (SPR). MMX-, MMF-, or MMXF-Tf were immoblized to CM5 sensor chips to yield a response of 4,000 to 8,000 RU. Purified human IgG and IgE in HBS-P buffer ran over the surface for 7 min, followed by another 7 min washing with protein-free buffer at a flow rate of 5 µL/min. Rabbit anti-HRP polyclonal serum (Sigma-Aldrich) was used as positive control. The affinity constants were determined by fitting to a single binding model by using BIAevaluation 3. Otherwise the procedures described in the previous studies have been followed. (Hantusch et al., 2005; Jin et al., 2006)

### IgG subclass determination

The ratio of IgG1+3 versus IgG4 in total and specific IgG was accomplished by mass-spectrometric procedure. In brief, the SDS-PAGE band of IgG was excised, digested with trypsin and analyzed by liquid chromatography-electrospray ionization-mass spectrometry (LC-ESI-MS) as described previously. The ratio of IgG4 in relation to the IgG1 (plus IgG3) was determined from the signal height of the peaks of the homologous peptides peptides ALPAPIEK (IgG1 & IgG3), GLPAPIEK (IgG2) and GLPSSIEK (IgG4); as well as SLSLSPG(+/-K) (IgG1, IgG2 & IgG3) and SLSLSLG(+/-K) (Ig-G4). In view of the very similar composition of these peptides, that peak heights faithfully reflect the molar ratios of the peptides and hence the IgG subclasses within the limits of experimental error.

### RESULTS

### Selection of CCD-positive sera

From the 170 sera investigated, 60 (35%) contained measurable amounts of anti-CCD IgE when tested in ELISA with HRP as CCD-allergen. 93 (54%) sera contained anti-CCD IgG. Noteworthy, there was a poor overlapping of these populations: 20 (12% of total or 33% of the IgE containing) sera contained only IgE but no IgG against CCDs. Compared to literature data, the prevalence of IgE-positivity is high but this is due to a deliberate bias in our selection of sera. The percentage of IgG-positive sera accords with recent data on individuals, which - in contrast to this work - had not been preselected by attending an allergy clinic. The numbers given above strongly depend on the chosen threshold levels for rating the ELISA results. Therefore, the results are shown as a plot of IgG versus IgE readings (Fig. 1). Apart from a few outlyers, the sera can be classified into three groups: (1) neither s-IgE nor s-IgG, (2) no s-IgE but s-IgG and (3) both s-IgE and s-IgG (Fig. 1). The patients with narrow sensitization contained almost no CCD-IgE but nevertheless many of them had s-IgG. The highest tendency for the occurrence of CCD-IgE was seen in insect venom double positive patients and in the "discrepant" group. This latter result impressively confirms the view that CCD epitopes lead to false-positive RAST results, which are not accompanied by positive SPTs nor, probably, by a real disposition for clinical symptoms. However, several sera in this group (about 28%) contained no CCD-IgE indicating that CCDs are not the only type of epitopes leading to false positive RAST results as also found in other cases. Additionally, also qualitative and quantitative differences in allergen content between the extracts used for CAP and skin testing may account for some of these discrepancies.

### Purification and specificity of CCD-binding human IgG and IgE

19 sera containing both IgG and IgE reacting with HRP, and hence presumably with CCDs, were pooled (final volume 20 mL) and subjected to the three stage affinity purification. Beside this, the specificities of antibodies in this serum pool were determined by Western blot (Fig. 2). The respective ELISA results were rather similar to what was found for the purified antibodies (see below). An interesting side-aspect was the reactivity of the pool's IgG and IgE with hemocyanin of *Helix pomatia* (Fig. 3), which corroborates the different specificities of human IgG and IgE as displayed by transferrin glycoforms. At the same time, this experiment also substantiates the usefulness of hemocyanin as a "xylose"-probe despite the complexity of its glycan structures.

Both IgG and IgE in the serum pool reacted equally well with MMXF and GnGnXF structures. The characterization of the human serum pool showed that IgE very specifically bound to glycoforms with core α1,3-fucose. In contrast, human IgG also recognized glycans with xylose but no fucose and thus displayed a broader specificity. However, IgG even bound to transferrin devoid of xylose or fucose (Fig. 2) and this "unspecific" binding turned out to perturb the following measurements by SPR. Therefore, the first purification step aimed at removal of IgG binding to unmodified transferrin (Fig. 4). The second affinity column contained glycopeptides coupled to BSA. In order to prevent loss of antibodies with narrow specificities, a mixture of glycoforms containing MMXF, MMF and MMX structures was used. The eluted, CCD-specific antibodies were now passed over protein G to separate IgG from IgE, which cannot bind to protein G (Fig. 4). The volumes and protein concentrations of the specific IgG and IgE fraction were 0.5 and 0.65 mL and 49.5 and 0.154 µg/mL (or 24.8 and 0.10 µg), respectively. Losses aside, this acounts for a about 250 fold molar excess of IgG over IgE. However, the determination of specific IgE and IgG in the serum pool before purification by ImmunoCAP with bromelain as a CCD-allergen yielded concentrations of 8.88 ng/mL and 11.4 µg/mL, respectively. This leads to a 1280 molar excess of IgG.

The specificity of the purified IgE resembled that from the serum pool whereas the purified IgG no longer bound to MM-glycoforms (Fig. 5). A noteworthy observation is the rather strong binding to glycoforms terminating with GlcNAc-residues (GnGnF, GnGnXF). This hitherto untested trait of human IgE contrasts earlier findings on commercial rabbit anti-HRP antibodies, where capping with GlcNAc drastically reduced antibody binding. However, rabbits can generate antibodies that are not kept off by terminal GlcNAc and apparently humans also have this ability. The antibodies have been purified over affinity columns with glycans devoid of terminal GlcNAc indicating that the same antibody population binds to both MMXF and GnGnXF glycans.

### Affinity of anti-CCD IgE and IgG

The binding affinity between CCD-reactive antibodies and their ligands was assessed by SPR using sensor chips with immobilized glycoforms of human transferrin. Using evaluation, the purified anti-MMXF IgG and IgE were applied to SPR analysis. The reference chip was coupled with unmodified transferrin. Various amounts of human anti-MMXF IgG and IgE were injected in triplicates onto the sensor chip. The "on-rate" *k*ₐ (M⁻¹s⁻¹) was determined from the association phase of the binding curve, whereas *k*_{d} (s⁻¹) was deduced from the dissociation phase. The results are summarized in Table 1. The binding to MMF- and MMXF-Tf was faster for IgE than for IgG resulting in lower affinity constants for IgE. Consistent with Western blots and ELISA results, human IgE did not bind to the MMX-Tf surface. IgG did bind to MMX-Tf, but the binding was of such a low affinity that the concentration of our human anti-CCD IgG preparation was insufficient for reaching a saturation plateau as required for calculation of the affinity constant. Thus, while some human IgG does bind to MMX or GnGnX structures, N-glycan xylosylation is an essentially irrelevant item for human allergy.

### SUMMARY OF THE RESULTS

Anti-CCD IgE antibodies, appear incapable of provoking clinical symptoms despite their frequent presence in serum. This empirical evidence implies that positive IgE tests must be rated as false-positive when they are solely based on CCD-reactivity. Anti-CCD-IgE can be detected by using glycoproteins, which are unlikely to contain peptideepitopes for human IgE such as pineapple stem bromelain or HRP. An even better choice might be artificial glyco-allergens. Inhibition experiments with such glyco-proteins can help to discriminate allergen binding via peptide or glycan epitopes. The more difficult question is on the reason for the biological insignificance.

As outlined in the introduction low valency of glyco-allergens hardly explains the lack of clinical manifestations.

A second explanation could come from binding affinity, which was judged to be low for glyco-determinants as seen from results of histamine release experiments. IgE-epitope affinity was shown to influence the efficieny of histamine release. Low affinity was also described as a reason for negative SPT results despite the presence of s-IgE. Therefore, our approach towards understanding the role of CCDs in allergy consisted in the determination of the binding affinity of purified human anti-CCD IgE. The stunningly low dissociation constants = i.e. high affinities of around 10⁻¹⁰ M for MMF- and MMXF-transferrin unmistakably rule out any deficiency of CCDs on this side.

The concentration of CCD-specific IgE in the serum pool was 3,7 kU/L (1.54 µg/mL), which is only RAST class II, but single sera with much higher titers of CCD-specific IgE are often found.

The affinity of CCD-specific IgG was determined to be about 100 times lower than that of IgE. This agrees at first sight with the results obtained for prominent allergens such as Bet v 1 and Phl p 5 (Table 2), or - measured by a method other than SPR - Amb a 5 . However, a closer look suggests the affinity of CCD-IgG to be comparably higher than that of anti-peptide IgG. While the IgE/IgG affinity ratio is about 2 orders of magnitude in the case of the CCD system, it is about 3 orders for Bet v 1 and Phl p 5 and even a bit higher for Amb a 5. The picture is considerable complicated by a report where the affinities of IgE, IgG1 and IgG4 have been determined in individual patients having or having not received birch pollen SIT. These authors rather found individual differences of affinity than systematic differences between IgE and the IgG subclasses.

**Table 2: Comparison of Binding affinities for antibodies to CCDs, Phl p 5 and Bet v 1.**

| Dissociation constants (M) for allergen: | | | | |
|---|---|---|---|---|
| | MMF | MMXF | PHL p 5a | Bet v 1 |
| IgE | 0.063 x 10⁻⁸ | 0.0068 x 10⁻⁸ | 0.028 x 10 ⁻⁸ | 0.0022 x 10 ⁻⁸ |
| IgG | 2.1 1 x 10⁻⁸ | 1.1 x 10⁻⁸ | 180 x 10⁻⁸ | 49 x 10⁻⁸ |
| ratio of affinities of IgE to IgG | 33 | 163 | 6430 | 2227 |

Notably, they observed that the degree of symptoms was correlated with the affinity of IgG1 and IgG4. Taken together, we hypothesize that the clinical insignificance of CCDs could be the joint result of the comparably high IgG affinity and a not too high titer of CCD-IgE. At this point it must be mentioned that the anti-CCD IgE titers in indivual patients may be up to ten times higher than that of our serum pool (Hemmer, unpublished results). It remains to be clarified whether such patients also respond asymptomatic towards glyco-allergens.

In the majority of patients, however, blocking antibodies of the IgG class appear to prevent clinical symptoms. This agrees with the ELISA results where patients with measurable s-IgE titers mostly also have CCD-specific IgG (Fig 1). The IgG may directly compete with IgE for allergen molecules. The product of relative quantity and affinity at least reaches the threshold where plain competition becomes imaginable. The predominance of IgG1 over IgG4 in our serum pool may harbour an argument against this explanation, as IgG4 is made responsible for direct inhibition. More recent work, however, revealed similar blocking activity for IgG4 and other IgG subclasses. A supporting or maybe even predominant mechanism might be antagonistic signalling via the tyrosine inhibition motif-containing immunore-ceptor FcγRIIb on mast cells and basophils upon binding of IgG allergen complexes. Other, cellular mechanism may also take place.

Repeated administration of high doses of an allergen has been amply demonstrated to lower early and late phase responses to that allergen, even though the mechanisms leading to tolerance are still under debate. We hypothesize that dietary uptake of plant glycoproteins effectuates mechanisms similar to those initiated by specific immunotherapy or, more precisely, by sublingual immune therapy (SLIT). Everyone is confronted life-long with food glyco-allergens in the gastrointestinal tract and by pollens on mucosal surfaces of the respiratory system. However, only around 20% of allergic individuals (see introduction) and an even smaller percentage of persons without allergic symptoms exhibit anti-CCD IgE. From the data of the current study and from another report, it can be concluded that many more people develop IgG against CCDs, but yet again not everyone. This raises the question on the onset and time course of the immune-reaction against CCDs.

The substantially higher affinity for structures with xylose in addition to the indispensable core α1,3-fucose may mean that plant glycoproteins play a vital role for induction and/or maturation of the IgE response. The initial trigger for the anti-CCD immune reaction, however, may be an insect sting. Short phase of allergic sensitization is soon followed by natural de-sensitization via incidential glyco-specific immunotherapy. The transient occurence of symptomatic allergic reactions towards CCD appears possible. Later, blocking antibodies would protect from symptoms and much later, s-IgE would gradually disappear - as in SLIT. It does not escape our attention that the mechanism proposed here for carbohydrate determinants could also apply to "mimickers of allergy" of protein nature. IgE against protein panallergens such as profilins is likewise known to be of clinical irrelevance in many cases (even though they may provoke skin reactions in SPT). These examples make a point for allergen resolved allergy diagnosis. More importantly for this example, they share with CCDs that patients are frequently and repeatedly exposed to them. Presumably, ubiquitous glyco-epitopes and panallergens probably loose their allergenic potency by the same mechanism.

Recently, regulatory T-cells have been described as the key element in allergen tolerance development. T_{Reg} cells and their products are thought to suppress activation of effector cells in an allergen-dependent manner. This model for allergen tolerance suggests that a tolerated allergen, say a glycoprotein with CCDs, would attenuate the reaction against a simultaneously present perilous allergen. Such an anti-allergic effect of CCDs has, however, not been described so far.

### EXAMPLE 2:

Efficiency of anti-CCD IgE removal from serum. Wasp venom was subjected to reducing SDS-PAGE to separate the three major allergens hyaluronidase (Hya), phospholipase (Pla) and antigen 5 (Ag5). Allergens were visualized by immunoblot with serum of wasp-allergic patient. Only hyaluronidase is glycosylated bearing N-glycans with core α1,3-linked fucose (Kolarich et al., FEBS J. 272 (2005): 5182-5190).
Lane 1 of Fig. 7 shows that the patient's serum binds to all three allergens.
Lane 2 of Fig. 7 was incubated with serum in the presence of an arteficial inhibitory glycoprotein (bromelain-glycopeptide coupled to bovine serum albumin) (Altmann Int. Arch. Allergy Immunol. 142 (2007): 99-115). The absence of a stained band at the position of hyaluronidase indicates that the serum only reacted with the carbohydrate moiety of hyaluronidase but not with the polypeptide as was the case for the phospholipase and antigen 5.
Lane 3 of Fig. 7 shows the immunoblot result with the same patient's serum after removal of the anti-CCD IgE using an affinity matrix according to the present invention.

## Claims

1. Method for removing cross-reactive carbohydrate determinant (CCD) specific antibodies from a sample comprising the steps of:
- providing a sample comprising or suspected to comprise CCD specific antibodies,
- contacting and incubating said sample with a solid support comprising at least one cross-reactive carbohydrate determinant, immobilised on its surface, and
- removing said solid support from the sample.

2. Method according to claim 1, **characterised in that** the sample comprises allergen specific antibodies.

3. Method according to claim 2, **characterised in that** the allergen specific antibodies are of the immunoglobulin isotype IgE, IgG and/or IgA.

4. Method according to claim 2 or 3, **characterised in that** the specificity and/or amount of the allergen specific antibodies in the sample is determined.

5. Method for diagnosing an allergy in an individual comprising the steps of:
- providing a sample of an individual,
- removing cross-reactive carbohydrate determinant (CCD) specific antibodies from said sample by contacting said sample and incubating said sample with a solid support comprising at least one cross-reactive carbohydrate determinant, immobilised on its surface, and removing said solid support from the sample,
- determining the presence and/or the amount of IgE specific for at least one allergen in the sample and
- diagnosing an allergy when the presence of allergen specific IgE is determined in the sample.

6. Method according to any one of claims 1 to 5, **characterised in that** the sample is of human or animal origin and preferably selected from the group consisting of whole blood, serum and plasma.

7. Method according to any one of claims 1 to 6, **characterised in that** the CCD is selected from the group consisting of β1,2-xylose and α1,3-fucose and is comprised in a glycan molecule, selected from the group consisting of (Manα1-3)Manα1-6(Xylβ1-2)Manβ1-4GlcNAcβ1-4GlcNAc (MMX), (Manα1-3)Manα1-6(Xylβ1-2)Manβ1-4GlcNAcβ1-4(Fucα1-3)GlcNAc (MMXF), Manα1-6(Xylβ1-2)Manβ1-4GlcNAcβ1-4(Fucα1-3)GlcNAc (MUXF), Manα1-6(Manα1-3)Manβ1-4GlcNAcβ1-4 (Fucα1-3)GlcNAc(MMF), Manα1-6Manβ1-4GlcNAcβ1-4 (Fucα1-3)GlcNAc (MUF), GlcNAcβ1-2Manα1-6(GlcNAcβ1-2Manα1-3)(Xylβ1-2)Manβ1-4GlcNAcβ1-4(Fucα1-3)GlcNAc (GnGnXF), GlcNAcβ1-2Manα1-6 (Manα1-3)(Xylβ1-2)Manβ1-4GlcNAcβ1-4(Fucα1-3)GlcNAc (GnMXF), Manα1-6(GlcNAcβ1-2Mancrl-3) (Xylβ1-2)Manβ1-4GlcNAcβ1-4(Fucα1-3)GlcNAc (MGnXF), GlcNAcβ1-4Manα1-6(Manα1-3)Manβ1-4GlcNAcβ1-4(Fucα1-3)GlcNAc (GnMF), Manα1-6(GlcNAcβ1-2Manα1-3)GlcNAcβ1-4(Fucα1-3)GlcNAc (MGnF) or GlcNAcβ1-2Manα1-6(GlcNAcβ1-2Manα1-3)Manα1-4GlcNAcβ1-4(Fucα1-3)GlcNAc (GnGnF).

8. Method according to any one of claims 1 to 7, **characterised in that** the CCD or glycan molecule is isolated from a plant or mammalian glycoprotein.

9. Method according to claim 8, **characterised in that** the plant glycoprotein is obtained from bromelain or patatin.

10. Method according to claim 8, **characterised in that** the mammalian glycoprotein or protein is obtained from fibrin and/or other vertebrate glycoproteins containing a substantial amount of diantennary N-glycans (e.g. serotransferrin, protease-inhibitors, immunoglobulins or egg yolk glycoproteins) is preferably modified with glycosidases or glycosyl transferases.

11. Method according to any one of claims 1 to 10, **characterised in that** the solid support is a functionalised or activated cellulose, agarose, dextran, poly-methacrylate, polystyrol, polyacrylamide or silica.

12. Method according to any one of claims 1 to 11, **characterised in that** said solid support is contained in a cartridge, filter or pipette tip or packed in a column, preferably chromatographic column.

13. Use of a solid support comprising at least one cross-reactive carbohydrate determinant, immobilised on its surface, in diagnosing an allergen sensitivity.

14. Kit for diagnosing an allergen sensitivity comprising:
- a solid support comprising at least one cross-reactive carbohydrate determinant, immobilised on its surface,
- means for determining the presence of allergen specific antibodies.

15. Kit according to claim 14, **characterised in that** the means for determining the presence of allergen specific antibodies comprise an allergen and means for detecting and optionally quantifying the binding of the allergen specific antibodies to the allergen.

16. Kit according to claims 14 or 15, **characterised in that** the solid support is contained in a cartridge, filter or pipette tip or packed in a column, preferably chromatographic column.
